(19) **Europäisches Patentamt**
**European Patent Office**
**Office européen des brevets**

(11) **EP 3 544 585 B1**

(12) **EUROPEAN PATENT SPECIFICATION**

(45) Date of publication and mention
of the grant of the patent:
**25.02.2026  Bulletin 2026/09**

(51) International Patent Classification (IPC):
*A61K 9/00* (2006.01)  *A61K 9/20* (2006.01)
*A61K 9/28* (2006.01)  *A61K 31/167* (2006.01)

(21) Application number: **17808734.2**

(52) Cooperative Patent Classification (CPC):
**A61K 9/2095; A61K 9/0056; A61K 9/284;**
**A61K 31/167**

(22) Date of filing: **27.11.2017**

(86) International application number:
**PCT/US2017/063282**

(87) International publication number:
**WO 2018/098434 (31.05.2018 Gazette 2018/22)**

(54) **PROCESS FOR MAKING A COATED DOSAGE FORM**

VERFAHREN ZUR HERSTELLUNG EINER BESCHICHTETEN DARREICHUNGSFORM

PROCÉDÉ DE FABRICATION D'UNE FORME POSOLOGIQUE REVÊTUE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **28.11.2016  US 201662426754 P**

(43) Date of publication of application:
**02.10.2019  Bulletin 2019/40**

(73) Proprietor: **Kenvue Brands LLC**
**Summit, NJ 07901 (US)**

(72) Inventors:
• **MCNALLY, Gerard P.**
**Fort Washington, Pennsylvania 19034 (US)**

• **ANDERSON, Oliver**
**Fort Washington, Pennsylvania 19034 (US)**
• **KOLL, Greg**
**Fort Washington, Pennsylvania 19034 (US)**
• **SZYMZCAK, Christopher**
**Fort Washington, Pennsylvania 19034 (US)**
• **PANDEY, Anurag**
**Fort Washington, Pennsylvania 19034 (US)**

(74) Representative: **Carpmaels & Ransford LLP**
**One Southampton Row**
**London WC1B 5HA (GB)**

(56) References cited:
**WO-A1-2015/105992    US-A1- 2011 070 286**
**US-A1- 2013 295 174    US-A1- 2013 295 211**

**Description**

BACKGROUND OF THE INVENTION

Pharmaceuticals intended for oral administration are typically provided in tablet form. Tablets are swallowed whole, chewed in the mouth, or disintegrated in the oral cavity. Soft tablets that either are chewed or dissolve in the mouth are often employed in the administration of pharmaceuticals where it is impractical to provide a tablet for swallowing whole. With chewable tablets, the act of chewing helps to break up the tablet particles as the tablet disintegrates and may increase the rate of absorption by the digestive tract. Soft tablets are also advantageous where it is desirable to make a pharmaceutically active agent available topically in the mouth or throat for both local effects or systemic absorption. Soft tablets are also utilized to improve drug administration in pediatric and geriatric patients. Soft tablets designed to disintegrate in the mouth prior to swallowing are particularly useful for improving compliance of pediatric patients.

Generally, soft tablets are made by compaction of a blend of powder ingredients and typically include a pharmaceutically active agent, flavoring, and/or binders. The powder blend is typically fed into the cavity of a die of a tablet press and a tablet is formed by applying pressure. Hardness of the resulting tablet is a direct function of the compaction pressure employed and the compatibility of the ingredients in the formulation. A softer tablet, having an easier bite-through, may be prepared by employing reduced compaction pressures. The resulting tablet is softer, but also more fragile, brittle, and easily chipped and disadvantageously can involve complex and costly processing steps. Examples of soft tablets designed to disintegrate in the mouth without chewing are disclosed in U.S. Patent Nos. 5,464,632, 5,223,264, 5,178,878, 6,589,554, and 6,224,905.

One approach to addressing the issues of fragility, brittleness, and chipping with softer tablets is to apply a coating to at least a portion of the tablet. The present invention relates to a process for making dosage forms, such as swallowable, chewable or orally disintegrating tablets, using radiofrequency energy ("RF energy") that includes one or more coating layers. US 2013/295174 A1 relates to a tablet including a first region and a second region, wherein: (i) the first region and the second region each include at least 10%, by volume, of the tablet; (ii) the first region includes a pharmaceutically active agent and the composition of the first region is different from the composition of the second region; (iii) the first region has a density less than about 0.8 g/cc; and (iv) the first region disintegrates in the mouth when placed on the tongue in less than about 30 seconds; wherein the shape of the tablet includes two opposing major faces separated by a side wall, and the interface between the first region and the second region is along at least one major face of the tablet.

US 2011/070286 A1 relates to a process for making a nicotine-comprising chewing gum by (i) dispensing a powder portion from a gum-base-comprising powder, (ii) optionally shaping said powder portion into a powder aggregate, and (iii) applying sufficient electromagnetic energy (EM energy) to said powder portion or said powder aggregate to transform said powder portion or said powder aggregate into said chewing gum, whereby said EM energy is Radio Frequency (RF) energy, MicroWave (MW) energy, InfraRed (IR) energy or UltraViolet (UV) energy or combinations thereof.

US 2013/295211 A1 relates to a machine for the production of a solid dosage form wherein the machine is adapted to form a dosage form between a first forming tool and a second forming tool within a forming cavity and wherein the first RF electrode and the second RF electrode are arranged within the machine such that when RF energy is applied between the first RF electrode and the second RF electrode, the RF energy passes through the portion of the forming cavity adapted to form the dosage form.

WO 2015/105992 A1 relates to a process for making a tablet comprising at least one pharmaceutically active agent, said method comprising the step of applying radiofrequency energy to a powder blend to sinter said powder blend into said tablet, wherein said powder blend comprises lossy coated particles and said at least one pharmaceutically active agent, wherein said lossy coated particles comprises a substrate that is at least partially coated with a lossy coating comprising at least one activator, wherein said substrate has a Q value of greater than 100 and said activator has a Q value of less than 75.

SUMMARY OF THE INVENTION

The present invention relates to a process for making a solid dosage form comprising at least one pharmaceutically active ingredient, the method comprising the steps of:

> (i) providing a shape of said dosage form comprising a first powder layer comprising a surface stabilizing agent, a second powder layer comprising at least one activator and the pharmaceutically active ingredient, and a third layer comprising a second surface stabilizing agent, wherein the second layer is between the first layer and the third layer;

> > wherein, when measured according to the Slotted Line Method defined in the description, the activator has a Q value of less than 50 and the surface stabilizing agents each have a Q value of greater than 65; wherein the Q value is defined by the equation:

$$Q \text{ value} = e' / e''$$

where e" is the dielectric loss and e' is the dielectric constant;
(ii) applying radiofrequency energy for a sufficient period of time to the shape of step (i); and
(iii) applying heat for a sufficient period of time to the shape of step (i) to melt or soften the surface stabilizing agents.

Step (ii) and step (iii) may be performed simultaneously, step (ii) may be performed before step (iii), or step (iii) may be performed before step (ii).

The present invention also relates to a process for making a dosage form comprising at least one pharmaceutically active ingredient, the method comprising the steps of:

(i) introducing a first powder layer comprising a surface stabilizing agent, a second powder layer comprising at least one activator and the pharmaceutically active ingredient, and a third layer comprising a second surface stabilizing agent, wherein the second layer is between the first layer and the third layer, into a forming cavity within a die platen,

wherein, when measured according to the Slotted Line Method defined in the description, the activator has a Q value of less than 50, and the surface stabilizing agents each have a Q value of greater than 65; wherein the Q value is defined by the equation:

$$Q \text{ value} = e' / e''$$

where e" is the dielectric loss and e' is the dielectric constant;

(ii) compacting the first powder layer, second powder layer and third powder layer by introducing at least one forming tool into the die platen with sufficient force such that the shape of the dosage form is formed;
(iii) applying a radiofrequency energy for a sufficient period of time to the shape of step (ii);
(iv) applying heat for a sufficient period of time to the shape of step (ii) to melt or soften the surface stabilizing agents; and
(v) removing the dosage form from the forming cavity.

Step (iii) and step (iv) may be performed simultaneously, step (iii) may be performed before step (iv), or step (iv) may be performed before step (iii).

DETAILED DESCRIPTION OF THE INVENTION

It is believed that one skilled in the art can, based upon the description herein, utilize the present invention to its fullest extent. The following specific embodiments can be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. As used herein, all percentages are by weight unless otherwise specified.

The present invention relates to a process for making a dosage form comprising at least one pharmaceutically active ingredient by applying radiofrequency. Examples of dosage forms include: tablets, such as swallowable, chewable, and orally disintegrating tablets; lozenges; and gums. Examples of the manufacture of such dosage forms are disclosed in US Patent Nos. 8871263, 9610224, 8807979, 8343533, 8784781, 8858210, 9233491, 9445971, 9511028, and 9789066 and in US Patent Application Nos. 20130295174, 20110070286, 20110318411, and 20110319492.

Q Value

The property of permittivity is the measure of the resistance to forming an electric field. For purposes of comparing materials in air, it is often convenient to describe the permittivity of material in air where the permittivity is more specifically called "relative permittivity" or $\epsilon_r$. This is a complex number represented by the following equation:

$$\epsilon_r = e' - je''$$

where e' (the real portion of the complex number) is the dielectric constant (energy storage) and e" (the imaginary portion of the complex number) is the dielectric loss or dissipation factor (energy dissipated as heat). The ratio of dielectric loss (e") over the dielectric constant (e') is called the loss tangent (tan $\delta$) or power factor. Since loss tangent values for materials used in foods/pharmaceuticals are very low at 27MHz, it is convenient to use the reciprocal of loss tangent or "Q value" hence,

$$Q \text{ value} = e' / e"$$

For purposes of this invention, the Q value is calculated for the frequency that the material is to be processed (e.g., 27 MHz). The Q value is affected by physical and chemical properties such as density (porosity/particle size), moisture (conductivity), temperature, and molecular polarizability. The measurements obtained by this method can eliminate the need to measure and evaluate these properties independently.

As the Q value becomes smaller, a material will heat more readily when an external electromagnetic field is applied.

For purposes of describing the components of the invention, a material which has a high Q value (e.g., which responds less to the external field) is referred to as a "passivator." Passivators can serve to insulate or impede energy flow. Conversely, lower Q values (e.g., having higher flux) are termed "activators," as energy is allowed to flow through more easily and do more work. For purposes of describing the present invention, passivators have Q values greater than about 100 (or greater than 200 or greater than 300), while activators have Q values less than 75 (such as less than 50).

The Q value for various materials is recited below in Table 1a. The Q values were measured using a HP 805C Slotted Line as sample holder (carriage removed) connected to Agilent N5230C PNA-L to ports A and B (transmission mode). The sample holder and coaxial wires with N-type connectors are calibrated at room temperature using 8592-60008 E-cal to eliminate error in loss measurement encountered from the coax lines/sample holder itself. This process is referred to as the "Slotted Line Method." Unless otherwise stated, the Slotted line method was used for the calculation of e', e", and Q value herein with the frequency range set to 26-28 MHz (31 points resolution) and values of e' and e" were recorded from the 27 MHz data point.

Table 1a

| Material | Function | e' | e" | Loss Tangent | Q Value |
|---|---|---|---|---|---|
| Hydroxyethyl cellulose (Natrosol L250), LOD=4.1% | activator | 2.0937 | 0.1574 | 0.0752 | 13 |
| Hydroxypropyl cellulose (Klucel EF), LOD=2.3% | activator | 1.7225 | 0.0972 | 0.0564 | 18 |
| Hydroxypropyl cellulose (Klucel ELF), LOD=1.6% | activator | 1.6404 | 0.0769 | 0.0469 | 21 |
| Hydroxypropyl cellulose (Klucel LF), LOD=1.8% | activator | 1.5964 | 0.0723 | 0.0453 | 22 |
| Hydroxypropyl cellulose (Klucel JF), LOD=1.8% | activator | 1.6248 | 0.0739 | 0.0455 | 22 |
| Hydroxypropyl cellulose (SSL-SFP) | activator | 1.4174 | 0.0520 | 0.0367 | 27 |
| Sucrose (Granular Table Sugar) | substrate | 1.9314 | 0.0096 | 0.0050 | 201 |
| Acetaminophen Coated with Ethylcellulose* | substrate | 1.8625 | 0.0050 | 0.0027 | 373 |
| Maltitol (SweetPearl DC300) | substrate | 1.6214 | 0.0035 | 0.0022 | 463 |
| *Note -taste-masked particle has coating but the coating does not have an activator (i.e., the Q value of ethylcellulose was measured to be 98). | | | | | |

Surface Stabilization Agent

A critical component of the first powder layer is a surface stabilization agent. When heat is applied, the surface stabilization agent melts or softens and binds the other components together to form a unified layer. The unified layer improves the structural integrity of the dosage form making it less brittle and more resistant against chipping.

Key material property considerations in selecting the surface stabilization agent include for example, (i) melting point, (ii) glass transition temperature, (iii) Q value, (iv) water dispersibility, and/or (v) water solubility. The selection of a surface stabilization agent is based on considering each material property. No one property is dispositive in its selection, but rather a combination of (i)-(v) is evaluated to determine a suitable surface stabilization agent.

In one embodiment, the surface stabilization agent has a melting point of from about 40 °C to about 200 °C.

In one embodiment, the glass transition temperature (Tg) of the surface stabilization agent is less than 150 °C. In another embodiment, the Tg of the first powder layer is at least 5% lower than the Tg of the second powder layer.

It is desirable that the Q value of the surface stabilization agent be greater than 65. Materials with a Q value greater than 65 are somewhat or not significantly affected when radiofrequency energy is applied. In one embodiment, the Q value of the surface stabilization agent is from about 65 to about 2200. It should be pointed out that in embodiments where the surface stabilization agent is comprised of more than one component, the dielectric property, e.g., Q value, of the surface stabilization agents will be determined by measuring the dielectric property of the mixture of components.

In one embodiment, the surface stabilizing agent and/or first powder layer and/or the third powder layer is water soluble. In these embodiments, the water solubility can be described as a function of the Hildebrand/Hansen solubility parameter, ($MPa^{1/2} = \delta$) which is described as a numerical estimate of the degree of interaction between materials, and wherein the solubility parameter of water is about 48 $MPa^{1/2}$. In one embodiment, the Hildebrand/Hansen solubility parameter of the surface stabilization agent of the first powder layer and/or the first powder layer and/or third powder layer is between about 19 and about 48 $MPa^{1/2}$.

In another embodiment, the first powder layer and/or third powder layer is water dispersible. In this embodiment, the first powder layer and/or third powder layer is water dispersable because it breaks apart in an aqueous medium upon contact, but the materials do not necessarily dissolve into solution. Desirably, the rate of dispersion of the first powder layer and/or third powder layer in water is not less than 5 seconds more than the second powder layer. Preferably, not less than 3 seconds more than the second powder layer.

In one embodiment, the surface stabilization agent is a polymer or a waxy material.

Non-limiting examples of surface stabilizing agents and their material properties are provided in Table 1b.

Table 1b

| Materials and Material Properties for Surface Stabilizing Agent | | | | | | |
|---|---|---|---|---|---|---|
| Material | $MPa^{1/2}$ (solubility parameter) | Loss on Drying @ 105°C | Water Activity | e' | e" | Q value |
| Polyvinyl caprolactam polyvinyl acetate-polyethylene glycol graft copolymer (Soluplus®) | 19.4 | 2.088 | 0.2761 | 2.040 | 0.0314 | 65 |
| Polyethylene Glycol 3350 (Carbowax®) | 22 | n/a | --- | 1.9532 | 0.0196 | 100 |
| Micronized Crospovidone (Kollidon® CLM) | | 9.201 | 0.3579 | 1.337 | 0.012 | 112 |
| Vinylpyrrolidone-vinyl acetate copolymer (Kollidon® VA64) | 21.2 | 4.937 | 0.3905 | 1.462 | 0.011 | 137 |
| Vinylpyrrolidone-vinyl acetate copolymer (Kollidon® VA64 fine) | 21.2 | 4.937 | 0.3905 | 1.2695 | 0.0053 | 240 |
| Copovidone (Plasdone® S-630) | 19.01 | 3.748 | 0.284 | 1.477 | 0.010 | 154 |
| Xylitol (Xylitab® 100) | 36.86 | 0.035 | 0.453 | 1.751 | 0.010 | 177 |
| Carbomer (Carbopol® 934) | 33 | 4.786 | 0.3859 | 1.256 | 0.003 | 419 |
| Precirol ATO 5 | --- | n/a | 0.1794 | 1.4721 | 0.0027 | 545 |
| Polyethylene Oxide (Polyox®WSR) | 19 | 0.131 | 0.4724 | 1.780 | 0.005 | 396 |
| Trehalose | Est. >30, Soluble in Water | 0.137 | 0.1935 | 1.5185 | 0.0007 | 2169 |
| Carbowax® & Polyox® - Commercially available from the Dow Corporation  Kollidon® & Soluplus® - Commercially available from the BASF Corporation  Plasdone® - Commercially available from the Ashland Corporation  Xylitab® - Commercially available from the Dupont Corporation  Carbopol® - Commercially available from Lubrizol Corporation  Precirol® - Commercially available from the Gattefosse Corporation  $MPa^{1/2}$: Hildebrand/Hansen Solubility parameter as a measure of water solubility | | | | | | |

In one embodiment, the surface stabilizing agent is vinylpyrrolidone-vinyl acetate copolymer. In another embodiment, the surface stabilizing agent is Precirol® ATO, which is composed of mono-, di and triglycerides of palmitostearic acid.

First and Third Powder Layers

In one embodiment, the surface stabilizing agent is at least about 50% by weight of the coating (weight percent of the first powder layer and third powder layer). In one embodiment, at least about 70% by weight of the coating. In another embodiment, the first powder layer comprises up to 20 %, for example up to 30% of a material with a Q value greater than 75, by weight of the first powder layer. In a version of this embodiment, this material may be a plasticizer, a sugar alcohol, a colorant, sweetener, a flow aid, a flavor or mixtures thereof.

The dosage form has a third powder layer which includes a second surface stabilizing agent (which may be same or different from the surface stabilizing agent in the first layer). In one embodiment, the third powder layer is comprised of components similar to the first powder layer and has attributes similar to the first powder layer. The second powder layer is positioned between the first powder layer and the third powder layer.

In one embodiment, the powder that makes up the first particle layer, and the third powder layer, has an average particle size of from 38 to 150 microns, such as from 88 to 105 microns, such as from about 45 to 75 microns.

The first powder layer and the third powder layer may be the same color or a different color from one another.

In one embodiment, letters, numbers, indicia, and combinations thereof are applied, etched, or laser ablated on or from the heat treated first powder layer. In another embodiment, letters, numbers, indicia, and combinations thereof are applied, etched, or laser ablated on or from the heat treated third powder layer.

The first layer (and third layer) forms a coating portion of the dosage form. In one embodiment, this coating is at least about 0.1% by weight of the dosage form. In one embodiment, the coating is from about 0.2% to about 10% by weight of the dosage form. In another embodiment, the coating is from about 0.5% to about 4% by weight of the dosage form. In still another embodiment, from about 0.25% to about 2% by weight of the dosage form.

After heat is applied, in one embodiment, the first and/or third powder layers have a thickness of about 2 microns to about 100 microns. In one embodiment, the thickness of the first and/or powder layer after heat is applied is from about 10 microns to about 50 microns.

The coating portion may also include a plasticizer. In one embodiment, from about 1 percent to about 40 percent of a plasticizer is included. In another embodiment, the plasticizer is from about 5 percent to about 20 percent by weight of the coating.

Moreover, in one embodiment, the Tg of the plasticizer combined with the surface stabilizing agent is less than 150 °C.

Suitable plasticizers include, but are not limited to propylene glycol, polyethylene glycol triethyl citrate, glycerin, triacetin, tributyl citrate, poloxamer, and mixtures thereof. Suitable poloxamers for use as plasticizers also include Poloxamer 188 and Poloxamer 407. In certain embodiments, materials may be added to improve flow or to fill in spaces in the surface of the second powder layer as part of the blend of the first powder layer. These materials may include silica, colloidal silica, sugars, sugar alcohols such as sorbitol, mannitol or maltitol, or starches such as maize starch, derivatized starch or pea starch, polyvinyl alcohol, polyvinylalcohol-polyethylene glycol co-polymers, carnauba wax, candelila wax, microcrystalline wax, medium chain triglycerides, glyceryl palmitostearate, and glyceryl behenate. These materials may be added at quantities up to 20% by weight of the first powder and/or third powder layers.

Additionally, any coloring agent suitable for use in a food or pharmaceutical products may be used in the present inventive composition. Typical coloring agents include, for example, azo dyes, quinopthalone dyes, triphenylmethane dyes, xanthene dyes, indigoid dyes, iron oxides, iron hydroxides, titanium dioxide, natural dyes, and mixtures thereof. More specifically, suitable colorants include, but are not limited to patent blue V, acid brilliant green BS, red 2G, azorubine, ponceau 4R, amaranth, D&C red 33, D&C red 22, D&C red 26, D&C red 28, D&C yellow 10, FD&C yellow 5, FD&C yellow 6, FD&C red 3, FD&C red 40, FD&C blue 1, FD&C blue 2, FD&C green 3, brilliant black BN, carbon black, iron oxide black, iron oxide red, iron oxide yellow, titanium dioxide, riboflavin, carotenes, anthocyanines, turmeric, cochineal extract, chlorophyllin, canthaxanthin, caramel, betanin, and mixtures thereof.

Similarly, a flavor may be included in the first, second, or third layer of the dosage form. The amount of flavor added will be dependent upon the desired taste characteristics. Additionally, a sweetener may be added to the first and third layer. Suitable sweeteners may include high intensity sweeteners including but not limited to sucralose, aspartame, stevia, or acesulfame K. In one embodiment, the percent by weight of the sweetener in the first and third layers is at least 5% greater than the percent by weight of the sweetener in the second layer.

Second Powder Layer

The present invention features a second powder layer comprising at least one activator and a pharmaceutically active ingredient. The activator allows for controlled heating of the second powder layer for the manufacture of the sintered dosage form. In one embodiment, the second powder layer comprises particles that include a substrate and the at least one activator. Preferably, the substrate has a Q value of greater than 100. The activator has a Q value of less than 50.

In one embodiment, the particles in the second powder layer are lossy coated particles comprising a substrate that is at least partially coated with a lossy coating comprising at least one activator. Examples of the manufacture of such particles and/or the manufacture of such dosage forms containing such particles are disclosed in US Patent Nos. 8871263, 9610224, 8807979, 8343533, 8784781, 8858210, 9233491, 9445971, 9511028, and 9789066 and in US Patent Application Nos. 20130295174, 20110070286, 20110318411, and 20110319492.

Methods of manufacturing such lossy coated particles include, but are not limited to, top spray coating, top spray granulation, wurster coating, rotor coating, high shear granulation, spray drying, spray congealing, hot melt extrusion, microencapsulation, spinning disk coating, and extrusion/ spheronization. In one embodiment, the coating material is dissolved into solution and sprayed onto the substrate. In another embodiment, the coating is blended with the substrate and water is added to the blend, utilizing processes such as high shear granulation or spray drying. In one embodiment, the coating solution is aqueous optionally containing other solvents.

In one embodiment, the activator is a cellulosic polymer. Suitable cellulosic polymers include but are not limited to, hydroxypropylcellulose, hydroxyethylcellulose, carboxymethylcellulose, methylcellulose, hypromellose, and mixtures thereof. Other suitable activators include polysaccharides and proteins such as starches, modified starches, gelling starches, and hydrocolloids; including but not limited to guar gum, carrageenan, maltodextrin, inulin, and, polyvinyl pyrrolidone. Still other suitable activators include acrylic polymers such as but not limited to: methacrylates such as polymethylmethacrylates; polyvinyls such as polyvinyl alcohols and polyvinyl acetates; and polycaprolactones. In another embodiment, the activator is hydrogenated starch hydrosylate. In one embodiment, the weight average molecular weight of the activator is less than 360,000 Daltons, such as less than 180,000 Daltons.

In one embodiment, the substrate (e.g., is the form of a particle) is comprised of materials selected from starches, sugars, sugar alcohols, dicalcium phosphate, and microcrystalline cellulose. Suitable sugars include but are not limited to sucrose, mannose, maltose, lactose, fructose, dextrose, and dextrose monohydrate. Suitable sugar alcohols include but are not limited to erythritol, sorbitol, xylitol, mannitol, and maltitol. In one embodiment, the substrate comprises the pharmaceutically active agent. In another embodiment, the substrate is coated with first coating prior to the addition of the lossy coating.

In one embodiment, the average particle size of the lossy coated particle is from about 50 to about 500 microns, such as from about 50 to about 400 microns, such as from about 50 to about 300 microns.

The lossy coated particle is at least partially coated with the coating. What is meant by at least partially coated is that at least 25% of the total surface area is covered with the coating, such as at least 50%, such as at least 75%, such as 100%. In one embodiment, the amount of activator(s) in the lossy coated particles is at least about 0.25%, by weight, of the lossy coated particles, such as at least about 0.4%, by weight. In one embodiment, the amount of activator(s) in the lossy coated particles is from about 0.1% to about 20%, by weight, of the lossy coated particles, such as from about 0.1% to about 10%, by weight, of the lossy coated particles, such as from about 0.1% to about 2%, by weight, of the lossy coated particles.

In one embodiment, the lossy coated particle contains water. In this embodiment, the lossy coated particle comprises at least 0.1 percent, by weight, water, such as at least 0.3 percent, by weight, water, such as at least 0.5 percent, by weight, water when measured using loss on drying at 105°C until the weight of the lossy coated particles has stabilized. In one embodiment, the lossy coated particle retains water when measured by loss on drying prior to sintering, such as moisture content of at least 0.1 percent by weight, such as from about 0.1 to about 3 percent, such as from about 0.5 to about 2 percent, by weight.

In one embodiment, the coating comprises more than one activator, such as two activators. In one embodiment, the coating comprises two polymers. In one embodiment, the coating comprises a plasticizer. Suitable plasticizers for include, but not be limited to: polyethylene glycol; propylene glycol; glycerin; sorbitol; triethyl citrate; tributyl citrate; dibutyl sebecate; vegetable oils such as castor oil, rape oil, olive oil, and sesame oil; surfactants such as polysorbates, sodium lauryl sulfates, and dioctyl-sodium sulfosuccinates; mono acetate of glycerol; diacetate of glycerol; triacetate of glycerol; natural gums; triacetin; acetyltributyl citrate; diethyloxalate; diethylmalate; diethyl fumarate; diethylmalonate; dioctylphthalate; dibutylsuccinate; glyceroltributyrate; hydrogenated castor oil; fatty acids; substituted triglycerides and glycerides.

In one embodiment, e' of the lossy coated particle (prior to blending) is at least 1.4, such as at least 1.6, such as 1.7 when measured at 27 MHz. In one embodiment, e" of the lossy coated particle (prior to blending) is at least 0.009, such as at least 0.015, such as at least 0.0300 when measured at 27 MHz.

Another method to measure Q value is by using an Agilent 4294A impedance analyzer using specially designed dielectric sample holder. The powder is filled in an empty puck by lightly and evenly pouring in the powder. The excess powder is leveled off to get a flat and even top surface. The first measurement is made by using a thin lid (1 mm) on the powder/puck. In subsequent measurements, the lid is removed and replaced with the next thicker lid. With each lid change, the thickness of the lid increases by 1 mm and the powder is further compressed. When the powder is fully compressed and the lid will not sit flush on the puck, the test is ended. The fully compressed powder along with the puck (without cap) is then weighed. The powder is then removed from the puck and the puck is thoroughly cleaned, to avoid cross contamination, and re-weighed empty to obtain a base weight before and after each different powder test. This allows the tests to be conducted at different powder density, and the tests can be performed at different temperatures, humidity and separate days. This process is referred to as the "Parallel Plate Method."

It has been discovered that coating a substrate comprising one or more passivators with a coating comprising one or more activators resulted in particles were surprising effective in a sintering process of forming very resilient dosage forms with fast disintegration. While not wanting to be bound by this theory, the synergy created by pre-bonding the activator(s) to the passivator (substrate) allows greater efficiency of bonding during sintering beyond simply additive effects.

In one embodiment, the substrate has a Q value of greater than 100, such as greater than 150, such as greater than 200, such as greater than 400. The activator has a Q value of less than 50, such as less than 30. In one embodiment, the lossy coated particle has a Q value of greater than 50, such as greater than 150, such as greater than 200. In one embodiment, the powder blend has a Q value of greater than 50, such as greater than 150, such as greater than 200.

Pharmaceutically Active Ingredient

The powder blend/dosage form of the present invention includes at least one pharmaceutically active agent containing particles. What is meant by a "pharmaceutically active agent" is an agent (e.g., a compound) that is permitted or approved by the U.S. Food and Drug Administration, European Medicines Agency, or any successor entity thereof, for the oral treatment of a condition or disease. Suitable pharmaceutically active agents include, but are not limited to, analgesics, anti-inflammatory agents, antipyretics, antihistamines, antibiotics (e.g., antibacterial, antiviral, and antifungal agents), antidepressants, antidiabetic agents, antispasmodics, appetite suppressants, bronchodilators, cardiovascular treating agents (e.g., statins), central nervous system treating agents, cough suppressants, decongestants, diuretics, expectorants, gastrointestinal treating agents, anesthetics, mucolytics, muscle relaxants, osteoporosis treating agents, stimulants, nicotine, and sedatives.

Examples of suitable gastrointestinal treating agents include, but are not limited to: antacids such as aluminum-containing pharmaceutically active agents (e.g., aluminum carbonate, aluminum hydroxide, dihydroxyaluminum sodium carbonate, and aluminum phosphate), bicarbonate-containing pharmaceutically active agents, bismuth-containing pharmaceutically active agents (e.g., bismuth aluminate, bismuth carbonate, bismuth subcarbonate, bismuth subgallate, and bismuth subnitrate), calcium-containing pharmaceutically active agents (e.g., calcium carbonate), glycine, magnesium-containing pharmaceutically active agents (e.g., magaldrate, magnesium aluminosilicates, magnesium carbonate, magnesium glycinate, magnesium hydroxide, magnesium oxide, and magnesium trisilicate), phosphate-containing pharmaceutically active agents (e.g., aluminum phosphate and calcium phosphate), potassium-containing pharmaceutically active agents (e.g., potassium bicarbonate), sodium-containing pharmaceutically active agents (e.g., sodium bicarbonate), and silicates; laxatives such as stool softeners (e.g., docusate) and stimulant laxatives (e.g., bisacodyl); H2 receptor antagonists, such as famotidine, ranitidine, cimetadine, and nizatidine; proton pump inhibitors such as omeprazole, dextansoprazole, esomeprazole, pantoprazole, rabeprazole, and lansoprazole; gastrointestinal cytoprotectives, such as sucraflate and misoprostol; gastrointestinal prokinetics such as prucalopride; antibiotics for H. pylori, such as clarithromycin, amoxicillin, tetracycline, and metronidazole; antidiarrheals, such as bismuth subsalicylate, kaolin, diphenoxylate, and loperamide; glycopyrrolate; analgesics, such as mesalamine; antiemetics such as ondansetron, cyclizine, diphenyhydroamine, dimenhydrinate, meclizine, promethazine, and hydroxyzine; probiotic bacteria including but not limited to lactobacilli; lactase; racecadotril; and antiflatulents such as polydimethylsiloxanes (e.g., dimethicone and simethicone, including those disclosed in United States Patent Nos. 4,906,478, 5,275,822, and 6,103,260); isomers thereof; and pharmaceutically acceptable salts and prodrugs (e.g., esters) thereof.

Examples of suitable analgesics, anti-inflammatories, and antipyretics include, but are not limited to, non-steroidal anti-inflammatory drugs (NSAIDs) such as propionic acid derivatives (e.g., ibuprofen, naproxen, ketoprofen, flurbiprofen, fenbufen, fenoprofen, indoprofen, ketoprofen, fluprofen, pirprofen, carprofen, oxaprozin, pranoprofen, and suprofen) and COX inhibitors such as celecoxib; acetaminophen; acetyl salicylic acid; acetic acid derivatives such as indomethacin, diclofenac, sulindac, and tolmetin; fenamic acid derivatives such as mefanamic acid, meclofenamic acid, and flufenamic acid; biphenylcarbodylic acid derivatives such as diflunisal and flufenisal; and oxicams such as piroxicam, sudoxicam, isoxicam, and meloxicam; isomers thereof; and pharmaceutically acceptable salts and prodrugs thereof.

Examples of antihistamines and decongestants, include, but are not limited to, bromopheniramine, chlorcyclizine, dexbrompheniramine, bromhexane, phenindamine, pheniramine, pyrilamine, thonzylamine, pripolidine, ephedrine, phenylephrine, pseudoephedrine, phenylpropanolamine, chlorpheniramine, dextromethorphan, diphenhydramine, doxylamine, astemizole, terfenadine, fexofenadine, naphazoline, oxymetazoline, montelukast, propylhexadrine, triprolidine, clemastine, acrivastine, promethazine, oxomemazine, mequitazine, buclizine, bromhexine, ketotifen, terfenadine, ebastine, oxatamide, xylomeazoline, loratadine, desloratadine, and cetirizine; isomers thereof; and pharmaceutically acceptable salts and esters thereof.

Examples of cough suppressants and expectorants include, but are not limited to, diphenhydramine, dextromethorphan, noscapine, clophedianol, menthol, benzonatate, ethylmorphone, codeine, acetylcysteine, carbocisteine, ambroxol, belladona alkaloids, sobrenol, guaiacol, and guaifenesin; isomers thereof; and pharmaceutically acceptable salts and prodrugs thereof.

Examples of muscle relaxants include, but are not limited to, cyclobenzaprine and chlorzoxazone metaxalone, orphenadrine, and methocarbamol; isomers thereof; and pharmaceutically acceptable salts and prodrugs thereof.

Examples of stimulants include, but are not limited to, caffeine.

Examples of sedatives include, but are not limited to sleep aids such as antihistamines (e.g., diphenhydramine), eszopiclone, and zolpidem, and pharmaceutically acceptable salts and prodrugs thereof.

Examples of appetite suppressants include, but are not limited to, phenylpropanolamine, phentermine, and diethylcathinone, and pharmaceutically acceptable salts and prodrugs thereof

Examples of anesthetics (e.g., for the treatment of sore throat) include, but are not limited to dyclonine, benzocaine, and pectin and pharmaceutically acceptable salts and prodrugs thereof.

Examples of suitable statins include but are not limited to atorvastin, rosuvastatin, fluvastatin, lovastatin, simvustatin, atorvastatin, pravastatin and pharmaceutically acceptable salts and prodrugs thereof.

In one embodiment, the pharmaceutically active agent included within the dosage form is selected from phenylephrine, dextromethorphan, pseudoephedrine, acetaminophen, cetirizine, aspirin, nicotine, ranitidine, ibuprofen, ketoprofen, loperamide, famotidine, calcium carbonate, simethicone, chlorpheniramine, methocarbomal, chlophedianol, ascorbic acid, pectin, dyclonine, benzocaine and menthol, and pharmaceutically acceptable salts and prodrugs thereof.

As discussed above, the pharmaceutically active agents of the present invention may also be present in the form of pharmaceutically acceptable salts, such as acidic/anionic or basic/cationic salts. Pharmaceutically acceptable acidic/anionic salts include, and are not limited to acetate, benzenesulfonate, benzoate, bicarbonate, bitartrate, bromide, calcium edetate, camsylate, carbonate, chloride, citrate, dihydrochloride, edetate, edisylate, estolate, esylate, fumarate, glyceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methylsulfate, mucate, napsylate, nitrate, pamoate, pantothenate, phosphate/diphospate, polygalacturonate, salicylate, stearate, subacetate, succinate, sulfate, tannate, tartrate, teoclate, tosylate and triethiodide. Pharmaceutically acceptable basic/cationic salts include, and are not limited to aluminum, benzathine, calcium, chloroprocaine, choline, diethanolamine, ethylenediamine, lithium, magnesium, meglumine, potassium, procaine, sodium and zinc.

As discussed above, the pharmaceutically active agents of the present invention may also be present in the form of prodrugs of the pharmaceutically active agents. In general, such prodrugs will be functional derivatives of the pharmaceutically active agent, which are readily convertible in vivo into the required pharmaceutically active agent. Conventional procedures for the selection and preparation of suitable prodrug derivatives are described, for example, in "Design of Prodrugs", ed. H. Bundgaard, Elsevier, 1985. In addition to salts, the invention provides the esters, amides, and other protected or derivatized forms of the described compounds.

Where the pharmaceutically active agents according to this invention have at least one chiral center, they may accordingly exist as enantiomers. Where the pharmaceutically active agents possess two or more chiral centers, they may additionally exist as diastereomers. It is to be understood that all such isomers and mixtures thereof are encompassed within the scope of the present invention. Furthermore, some of the crystalline forms for the pharmaceutically active agents may exist as polymorphs and as such are intended to be included in the present invention. In addition, some of the pharmaceutically active agents may form solvates with water (e.g., hydrates) or common organic solvents, and such solvates are also intended to be encompassed within the scope of this invention.

In one embodiment, the pharmaceutically active agent or agents are present in the dosage form in a therapeutically effective amount, which is an amount that produces the desired therapeutic response upon oral administration and can be readily determined by one skilled in the art. In determining such amounts, the particular pharmaceutically active agent being administered, the bioavailability characteristics of the pharmaceutically active agent, the dose regime, the age and weight of the patient, and other factors must be considered, as known in the art.

The pharmaceutically active agent may be present in various forms. For example, the pharmaceutically active agent may be dispersed at the molecular level, e.g. melted, within the dosage form, or may be in the form of particles, which in turn may be coated or uncoated. If the pharmaceutically active agent is in form of particles, the particles (whether coated or uncoated) typically have an average particle size of from about 1 to about 500 microns. In one embodiment, such particles are crystals having an average particle size of from about 1 to about 300 microns.

The pharmaceutically active agent may be present in pure crystal form or in a granulated form prior to the addition of the taste masking coating. Granulation techniques may be used to improve the flow characteristics or particle size of the pharmaceutically active agents to make it more suitable for subsequent coating. Suitable binders for making the granulation include but are not limited to starch, polyvinylpyrrolidone, polymethacrylates, hydroxypropylmethylcellulose, and hydroxypropylcellulose. The particles including pharmaceutically active agent(s) may be made by cogranulating the pharmaceutically active agent(s) with suitable substrate particles via any of the granulation methods known in the art. Examples of such granulation method include, but are not limited to, high sheer wet granulation and fluid bed granulation such as rotary fluid bed granulation.

If the pharmaceutically active agent has an objectionable taste, the pharmaceutically active agent may be coated with a taste masking coating, as known in the art. Examples of suitable taste masking coatings are described in U.S. Patent No. 4,851,226, U.S. Patent No. 5,075,114, and U.S. Patent No. 5,489,436. Commercially available taste masked pharmaceutically active agents may also be employed. For example, acetaminophen particles, which are encapsulated with ethylcellulose or other polymers by a coacervation process, may be used in the present invention. Coacervation-encapsulated acetaminophen may be purchased commercially from Eurand America, Inc. (Vandalia, Ohio).

**In** one embodiment, the dosage form incorporates modified release coated particles (e.g., particles containing at least one pharmaceutically active agent that convey modified release properties of such agent). As used herein, "modified release" shall apply to the altered release or dissolution of the active agent in a dissolution medium, such as gastrointestinal fluids. Types of modified release include, but are not limited to, sustained release or delayed release. In general, modified release dosage forms are formulated to make the active agents(s) available over an extended period of time after ingestion, which thereby allows for a reduction in dosing frequency compared to the dosing of the same active agent(s) in a conventional dosage form. Modified release dosage forms also permit the use of active agent combinations wherein the duration of one pharmaceutically active agent may differ from the duration of another pharmaceutically active agent. In one embodiment, the dosage form contains one pharmaceutically active agent that is released in an immediate release manner and an additional active agent or a second portion of the same active agent as the first that is modified release.

Examples of swellable, erodible hydrophilic materials for use as a release modifying excipient for use in the modified release coating include water swellable cellulose derivatives, polyalkylene glycols, thermoplastic polyalkylene oxides, acrylic polymers, hydrocolloids, clays, and gelling starches. Examples of water swellable cellulose derivatives include sodium carboxymethylcellulose, cross-linked hydroxypropylcellulose, hydroxypropyl cellulose (HPC), hydroxypropylmethylcellulose (HPMC), hydroxyisopropylcellulose, hydroxybutylcellulose, hydroxyphenylcellulose, hydroxyethylcellulose (HEC), hydroxypentylcellulose, hydroxypropylethylcellulose, hydroxypropylbutylcellulose, and hydroxypropylethylcellulose. Examples of polyalkylene glycols include polyethylene glycol. Examples of suitable thermoplastic polyalkylene oxides include poly (ethylene oxide). Examples of acrylic polymers include potassium methacrylatedivinylbenzene copolymer, polymethylmethacrylate, and high-molecular weight cross-linked acrylic acid homopolymers and copolymers.

Suitable pH-dependent polymers for use as release-modifying excipients for use in the modified release coating include: enteric cellulose derivatives such as hydroxypropyl methylcellulose phthalate, hydroxypropyl methylcellulose acetate succinate, and cellulose acetate phthalate; natural resins such as shellac and zein; enteric acetate derivatives such as polyvinylacetate phthalate, cellulose acetate phthalate, and acetaldehyde dimethylcellulose acetate; and enteric acrylate derivatives such as for example polymethacrylate-based polymers such as poly(methacrylic acid, methyl methacrylate) 1:2 (available from Rohm Pharma GmbH under the tradename EUDRAGIT S) and poly(methacrylic acid, methyl methacrylate) 1:1 (available from Rohm Pharma GmbH under the tradename EUDRAGIT L).

In one embodiment, the pharmaceutically active agent is coated with a combination of a water insoluble film forming polymer (such as but not limited to cellulose acetate or ethylcellulose) and a water-soluble polymer (such as but not limited to povidone, polymethacrylic co-polymers such as those sold under the tradename Eudragit E-100 from Rohm America, and hydroxypropylcellulose). In this embodiment, the ratio of water insoluble film forming polymer to water soluble polymer is from about 50 to about 95 percent of water insoluble polymer and from about 5 to about 50 percent of water soluble polymer, and the weight percent of the coating by weight of the coated taste-masked particle is from about 5 percent to about 40 percent.

In one embodiment, one or more pharmaceutically active agents or a portion of the pharmaceutically active agent may be bound to an ion exchange resin for the purposes of taste-masking the pharmaceutically active agent or delivering the active in a modified release manner.

In one embodiment, the pharmaceutically active agent is capable of dissolution upon contact with a fluid such as water, stomach acid, intestinal fluid or the like. In one embodiment, the dissolution of the dosage form containing the pharmaceutically active agent meets USP specifications for immediate release. For example, for acetaminophen tablets, USP 24 specifies that in pH 5.8 phosphate buffer, using USP apparatus 2 (paddles) at 50 rpm, at least 80% of the acetaminophen contained in the tablet is released there from within 30 minutes after dosing, and for ibuprofen tablets, USP 24 specifies that in pH 7.2 phosphate buffer, using USP apparatus 2 (paddles) at 50 rpm, at least 80% of the ibuprofen contained in the tablet is released there from within 60 minutes after dosing. See USP 24, 2000 Version, 19 - 20 and 856 (1999). In another embodiment, the dissolution characteristics of the pharmaceutically active agent are modified: e.g. controlled, sustained, extended, retarded, prolonged, delayed and the like.

In one embodiment, the pharmaceutically active agent(s) are comprised within polymer-coated particles (e.g., taste-masked and/or sustained release coated particles). In one embodiment, the pharmaceutically active ingredient is first coated with a taste-masking coating and subsequently coated with a second layer of a dielectric coating. In one embodiment, the pharmaceutically active agent(s) is included within the substrate and/or the coating layer of the lossy coated particle.

In one embodiment, the powder blend/dosage form comprises from about 10% to about 40%, by weight of the pharmaceutically active agents(s), such as 15% to about 35%, by weight of the dosage form/powder blend, such as 20% to about 30%, by weight of the dosage form/powder blend.

As discussed above, in one embodiment, the pharmaceutically active agent is or is comprised within the substrate of the lossy coated particles. In one embodiment, the amount of such coated particles comprising pharmaceutically active agents(s) may be present at level from about 10% to about 95%, by weight of the dosage form/powder blend, such as 15% to about 70%, by weight of the dosage form/powder blend, such as 20% to about 50%, by weight of the dosage form/powder blend.

In one embodiment, the pharmaceutically active agent(s) are comprised within lossy coated particles. In another embodiment, the pharmaceutically active ingredient is first coated with a taste-masking coating in absence of an activator and subsequently coated with a second layer containing an activator. In still another embodiment the pharmaceutically active ingredient is added to the outer coating layer containing an activator.

Forming the Dosage Form Shape

The composition of the first and/or third powder layers may be prepared prior to formation of the dosage form shape. This may be done to incorporate polymers, dyes, flavors, sweeteners and plasticizers into a uniform blended material which then, for example, can be added to the die cavity to form the dosage form shape prior to activation and heating. In one embodiment, this first powder (and optionally third powder) layer may be prepared by spray drying, spray congealing, melt extrusion, wet granulation, or dry granulation. In some embodiments, this blend may be sized or milled to provide a suitable particle size.

In one embodiment, to obtain desired attribute of an orally disintegrating tablet, the dosage form's construction may be highly porous and/or have a low density (e.g., to allow the tablet to collapse in the oral cavity). In a preferred embodiment, a minimum or no tamping is desired to achieve the orally disintegrating property.

In one embodiment, the tamping step (which occurs prior to the addition of the radiofrequency energy) applies a force to the cavities holding the powdered layers (e.g., material) to remove air from within the void space between particles and allows material to form into a shape. In one embodiment, the tamping step applies a force to the cavities holding three powdered layers. The force applied is less than about 450 pounds per square inch (e.g., less than about 300 pounds per square inch, such as less than 200 pounds per square inch, such as less than 50 pounds per square inch) which comes to rest on a frame (or mechanical "stop") preventing further deformation of material and without the RF energy no dosage form is formed. In one embodiment, the energy is applied while the powder blend is under such force without the use of a mechanical stop.

In one embodiment, the tamping step occurs in an indexed manner, where one set of dosage forms is processed simultaneously, before rotating to another indexing station. In one embodiment, the tamping step occurs at a single indexing station and the application of energy occurs at a separate indexing station. In another embodiment, a third indexing station is present wherein the ejection of the dosage form or multiple dosage forms occurs, wherein the lower forming tool is raised up through and up to the surface of the die. In another embodiment, the tamping step is performed through the addition of air pressure or hydraulic cylinder to the top of the upper forming tools. In one embodiment, multiple dosage forms are ejected simultaneously and separated from the surface of the indexing station and removed via a take-off bar.

In another embodiment, the dosage form shape may be prepared by methods and apparatus described in United States Patent Application Publication No. 20040156902. Specifically, the dosage form shape may be made using a rotary compression module including a fill zone, insertion zone, compression zone, ejection zone, and purge zone in a single apparatus having a double row die construction. The dies of the compression module may then be filled using the assistance of a vacuum, with filters located in or near each die. The purge zone of the compression module includes an optional powder blend recovery system to recover excess powder blend from the filters and return the powder blend to the dies.

In one embodiment, the dosage form shape is prepared by the methods and apparatus described in issued U.S. Patent No. 6,767,200. Specifically, the dosage form shape is made using a rotary compression module including a fill zone, compression zone, and ejection zone in a single apparatus having a double row die construction as shown in FIG. 6 therein. The dies of the compression module are preferably filled using the assistance of a vacuum, with filters located in or near each die.

The dosage form shape may have one of a variety of different shapes. For example, the dosage form shape may be shaped as a polyhedron, such as a cube, pyramid, prism, or the like; or may have the geometry of a space figure with some non-flat faces, such as a cone, truncated cone, triangle, cylinder, sphere, torus, or the like. In certain embodiments, a dosage form shape has one or more major faces. For example, the dosage form shape surface typically has opposing upper and lower faces formed by contact with the upper and lower forming tool faces (e.g., die punches). In such embodiments, the dosage form shape surface typically further includes a "belly-band" located between the upper and lower faces, and formed by contact with the die walls. Applicants have found that sharp edges in the tooling used to make the dosage forms can cause arcing, and thus more rounded edges may be needed.

In one embodiment, a vibratory step is utilized (e.g., added after filling of the powder blend but prior to the heating or fusing step, in order to remove air from the powder blend). In one embodiment, a vibration with the frequency from about 1 Hz to about 50 KHz is added with amplitude from 1 micron to 5 mm peak-to-peak to allow for the flowable powder blend to settle into the cavity of a die platen ("forming cavity").

Application of Radiofrequency Energy

The process includes the step of applying radiofrequency energy to the powder layers for a sufficient period of time to form such dosage form. While not wanting to be bound to any particular theory, it is believed that the pre-bonding of the activator on the surface of a passivator (the substrate) may provide a more direct path for the energy to travel due to higher conductivity at the surface. Such heating may be dielectric heating (e.g., using a lossy polymer containing vinyl, esters, amides, and/or urethane functional groups) or ionic heating. For ionic heating, as the field flows through the blend over the surface of the lossy coated particles, trapped moisture in the powder blend can provide a source of storing energy (e.g., at 27MHz, pure water has high dielectric constant) for the lossy coating. The higher loss polymer/activator can efficiently use the energy stored from the moisture to soften and flow the polymeric chains to form physical bonds through polymeric chain entanglement. The synergy provided by the configuration of the lossy coated particle can even provide enough bond strength to allow materials which do not provide a conductive path (or contain a lossy material) to be mixed into the lossy coated particle, where the invention serves as a filler.

Radiofrequency heating generally refers to heating with electromagnetic field at frequencies from about 1 MHz to about 100 MHz. In one embodiment of the present invention, the RF-energy is within the range of frequencies from about 1MHz to about 100MHz (e.g., from about 5MHz to 50MHz, such as from about 10MHz to about 30MHz). In one embodiment, the RF-energy is used to heat the first material. RF energy generators are well known in the art. Examples of suitable RF generators include, but are not limited to, free running oscillators such as the COSMOS Model C10X16G4 (Cosmos Electronic Machine Corporation, Farmingdale, NY) or a 50 Ohm RF generator. In one embodiment, the RF energy is combined with a second source of heat including but not limited to infrared, induction, or convection heating.

In the embodiment, the electrodes are incorporated into a chamber holding the powder blend (e.g., a cylinder, walled-sheet, or other chamber). In one embodiment, the chamber is constructed of a conductive metal. In one embodiment, the chamber has portions which are constructed of non-conductive, insulative material. In one embodiment, the chamber has an insert which is non-conductive where the body of the chamber is conductive. In one embodiment, the insert comprises a surface area which is less than that of the chamber. The conductive material may be comprised of any material which is conductive, including but not limited to aluminum, copper, iron, zinc, nickel and mixtures and alloys thereof. The non-conductive material may be comprised of a non-conductive solid material including but not limited to ceramics, polystyrene and polytetrafluoroethylene. In one embodiment, the chamber has at least one electrode embedded into the walls of the cylinder or walled sheet. The electrode may be surrounded by non-conductive material wherein the electrode is the only conductive wall portion exposed to the power blend. In one embodiment, the powder blend is tamped prior to the addition of RF-energy.

In one embodiment, one chamber contains the powder blend and it is placed into a separate chamber (e.g., an oven) for the addition of energy. In another embodiment, the chamber containing the powder blend has additional heating elements incorporated into the chamber.

After the application of energy, the powder blend may optionally be cooled (e.g., actively cooled or allowed to cool) prior to forming a predetermined amount of the energy-applied powder blend into the dosage form.

Examples of apparatuses useful for such application of energy are set forth in US Patent Applications Publ. Nos. 20110068511 and 20130295211.

Application of Heat

The process includes the step of applying heat to the first powder layer, and the third powder layer, for a sufficient period of time to form such dosage form. In one embodiment, the dosage form is treated with energy (e.g., convection or radiant heat energy) to soften or melt the surface stabilizing agent in the first powder layer, and the third powder layer, on the surface of the dosage form and then cooled or allowed to cool to further smooth the texture, enhance the gloss of surface of the dosage form, limit the friability of the dosage form, and/or provide a mark for identification. In one embodiment, the surface of the dosage form is exposed to infrared energy wherein the majority (at least 50 percent, such as least 90 percent, such as at least 99 percent) of the wavelength of such infrared energy is from about 0.5 to about 5 micrometers such as from about 0.8 to about 3.5 micrometers (e.g., by use of a wavelength filter). In one embodiment, the infrared energy source is a quartz lamp with a parabolic reflector (e.g., to intensify the energy) and a filter to remove unwanted frequencies. Examples of such radiant heat energy sources include the SPOT IR 4150 (commercially available from Research, Inc., Eden Prairie, MN). In one embodiment, the heat is applied to all the powder layers. It should be noted that the heat may be applied before or after removing the dosage form from the forming cavity (die platen).

In one embodiment, the temperature at which the heat is applied is at least 1°C, or at least 5°C, above the melting point and/or above the glass transition temperature of the surface stabilizing agent or the combination of the surface stabilizing agent and the plasticizer(s). In one embodiment, this heating step is applied while the dosage form shape is retained in the die. In another embodiment, the dosage form shape is ejected from the die cavity following the application of radiofrequency energy step, and the heating step is then applied to the dosage form shape.

Effervescent Couple

In one embodiment, the powder blend/dosage form further contains one or more effervescent couples. In one embodiment, effervescent couple contains one member from the group consisting of sodium bicarbonate, potassium bicarbonate, calcium carbonate, magnesium carbonate, and sodium carbonate, and one member selected from the group consisting of citric acid, malic acid, fumaric acid, tartaric acid, phosphoric acid, and alginic acid.

In one embodiment, the combined amount of the effervescent couple(s) in the powder blend/dosage form is from about 2 to about 20 percent by weight, such as from about 2 to about 10 percent by weight of the total weight of the powder blend/dosage form.

Orally Disintegrating Tablet

In one embodiment, the dosage form is a tablet designed to disintegrate in the mouth when placed on the tongue in less than about 60 seconds, e.g. less than about 45 seconds, e.g. less than about 30 seconds, e.g. less than about 15 seconds.

In one embodiment, the present invention features a process for making a tablet comprising at least one pharmaceutically active ingredient, the method comprising the steps of (i) introducing a first powder layer comprising a surface stabilizing agent according to the claims and a second powder layer comprising at least one activator according to the claims and the pharmaceutically active ingredient, and a third layer comprising a second surface stabilizing agent according to the claims, wherein the second layer is between the first layer and third layer, into a forming cavity within a die platen; (ii) compacting the first powder layer, second powder layer and third powder layer by introducing at least one forming tool into said die platen with sufficient force such that a tablet shape is formed; (iii) applying a radiofrequency energy for a sufficient period of time to the tablet shape of step (ii); and (iv) applying heat for a sufficient period of time to the tablet shape of step (ii) to melt or soften the surface stabilizing agents; and (v) removing the tablet from the forming cavity.

In one embodiment, the step of applying radiofrequency energy and the step of applying heat are performed simultaneously. In another embodiment, the step of applying radiofrequency energy is performed before the step of applying heat. In still another embodiment, the step of applying heat is performed before the step of applying radiofrequency energy.

Optionally, the process for making the tablet includes a cooling step, wherein the tablet is cooled in the die prior to its removal from the die.

In one embodiment, the tablet meets the criteria for Orally Disintegrating Tablets (ODTs) as defined by the draft Food and Drug Administration guidance, as published in April 2007. In one embodiment, the tablet meets a two-fold definition for orally disintegrating tablets including the following criteria: 1) that the solid tablet is one which contains medicinal substances and which disintegrates rapidly, usually within a matter of seconds, when placed upon the tongue and 2) be considered a solid oral preparation that disintegrates rapidly in the oral cavity, with an in vitro disintegration time of approximately 30 seconds or less, when based on the United States Pharmacopeia (USP 24 NF 29) disintegration test method for the specific medicinal substance or substances.

In one embodiment, the oral disintegration time of the tablet containing a coating is less than 30 seconds, for example less than 15 seconds, for example less than 10 seconds. In a version of this embodiment, the first layer, and if present the third powder layer, has an oral disintegration time that is within 10% of oral disintegration time of the second layer, or within 5 seconds of the second layer.

Hardness, Friability, and Density of Dosage Form

In one embodiment, the dosage form is prepared such that the dosage form is relatively soft (e.g., capable of disintegrating in the mouth or being chewed). The hardness test (crushing hardness) is based on hardness of the dosage form measured perpendicular to the cross-section at the belly band using a modified Model 6d, Pharmatron hardness tester fitted with a 50g force load cell (lower forces required for testing the invention). Unless otherwise indicated, testing is conducted on two stacked dosage forms, and the hardness is reported as 50% of the hardness measured. In one embodiment, the hardness of the dosage form is less than 1 kiloponds, such as less than 0.5 kiloponds.

In one embodiment, the density of the dosage form is at least about 0.6 g/cc. In one embodiment, the density of the dosage form is less than about 1.5 g/cc. In one embodiment, the bulk density of the lossy coated particles is from about 0.5 g/cc to about 1 g/cc.

In one embodiment, the dosage forms are tablets and have a friability of less than 10 percent, such as less than 5 percent, such as less than 3 percent. As used herein, "friability" is measured using the USP 24 NF 29 Tablet Friability (Section 1216) with the modification of using 3 tablets for 15 rotations or 3 tablets for 100 revolutions (unless otherwise noted) instead of 10 tablets for 100 rotations.

Use of Dosage form

The dosage forms may be used as swallowable, chewable, or orally disintegrating dosage forms to administer the pharmaceutically active agent.

Examples

Specific embodiments of the present invention are illustrated by way of the following examples. This invention was not confined to the specific limitations set forth in these examples.

**Example 1: Production of Lossy Coated particles with Acetaminophen and Maltitol Using Preparation of Hydroxyethylcellulose (HEC)**

Lossy Coating Solution:

1. Purified Water USP added to a suitably sized stainless steel container.
2. 132g of hydroxyethyl cellulose or HEC (commercially available as Natrosol® from Ashland Specialty Ingredients), as an activator, was added with 132g sodium citrate (to increase ionic strength) and 132g glycerin (a humectant/-plasticizer) make a concentration of 2.5% HEC in solution.

Coating of Substrate Particles with Hydroxyethyl Cellulose Coating Solution:

1. 7000 g of maltitol and 13000 g of taste-masked acetaminophen (previously coated with ethyl cellulose- a passivator, and commercially available from the Aptalis Corporation as Acetaminophen Microcaps®) was added to a fluid bed, top spray granulator.
2. The Lossy Coating Solution was sprayed onto the mixture of substrates at an approximate average spray rate of 134g/minute to make a 1.5% (w/w) lossy coated particle to a target end of spray percent moisture as noted in Table 2 as measured by loss on drying.
3. The granules were then dried to percent moisture as recorded in Table 3.

**Example 2: Production of Coating Material for Sintered Orally Disintegrating Tablet (sODT)**

Coating Material Slurry:

1. The materials outlined in Table 2 were added to a suitable size vessel.
2. 500g of water was added and mixed until dissolved.
3. The slurry was then placed into an oven at 50°C and allowed to dry
4. The dried film was then milled into a powder. Material particle size was between No. 140 and 170 US Mesh Screens.
5. 0-3 percent of Silicon dioxide was blended to mixture from step #4 immediately prior to use.

Table 2

| Coating Materials for Dry Coating | | |
|---|---|---|
| Material | % weight/weight | Batch Weight (g) |
| Kollidon® VA 64[1] | 87.0 | 87.0 |
| Sucralose NF | 5.0 | 5.0 |
| Flavor | 5.0 | 5.0 |
| Titanium Dioxide | 3.0 | 3.0 |
| TOTAL | 100.0 | 100.0 |
| 1: Kollidon® VA64 vinylpyrrlidone-vinyl actetate copolymer, commercially available from the BASF Corporation | | |

## Example 3: Preparation of Tablets

### Step A: Addition to Die and Tamping Step

The following materials were added in order to a placed into an electrically insulative Teflon, or ceramic die platen having a forming cavity that is approximately 1.1" in diameter and 0.175" thick. Approximately 8 mg of the coating material from Example 2 was added to the bottom of an insulated die.

Approximately 600mg of the substrate particles from Example 1 were then added. An additional 8mg of the coating materials from Example 2 was also added to the die for the top portion of the coating. The powder blend was then tamped between an upper and lower metal forming tools into a shape conformal to the surface of the forming tools. The tamping pressure was typically between 10 and approximately 100 psi of pressure.

### Step B: Radiofrequency Activation of Tablet

The forming tools, die platen and tablet shape were then placed between the upper RF electrode and lower RF electrode of an RF heating unit using a 50-ohm RF generator having an output of 4 KW of power and a frequency of 27 MHz. The upper RF electrode was brought into contact with the upper forming tool and the lower RF electrode is brought into contact with lower forming tool. The RF heating unit was energized for 1 second. After cooling, the resulting tablet was then ejected from the die platen using the lower forming tool.

### Step C: Heating of Surface

The ejected tablets from Step B are heated using the following steps:

1. Tablet is presented to a flat heated surface at 565.5°C (Maximum)
2. Tablet is not allowed to touch surface and is maintained at a minimum distance of approximately .06 - .125 inches.
3. Tablet is kept in position for approximately 2 - 3 second to transfer enough energy to begin bond on/within shell and substrate material. Surface temperatures may reach between 21 - 230°C)
4. Tablet is allowed to cool/solidify (undetermined period of time) and rotated to expose its opposite face; steps 1 - 3 are repeated.

The disintegration time as measured by USP 40-NF25 was less than 30 seconds and the friability of the tablets was less than 3 percent (15 drops of 3 tablets) using a friability apparatus outlined in USP 40-NF25.

Table 3

| Tablet Parameters | |
|---|---|
| Material/Output | Tablet Parameters |
| Particles | 0.5% HEC on Maltitol and Taste Masked Acetaminophen [lot 4094-078E] |
| Blend | 96.6% Maltitol and Coated Acetaminophen Particles with 3.34% flavors and adjuncts |
| Activator | HEC |

(continued)

| Tablet Parameters | |
| --- | --- |
| Material/Output | Tablet Parameters |
| Activator Concentration (%w/w) | 0.5 |
| Average Molecular Weight of Polymer (x1000 Daltons) | conforms to USP |
| Pharmaceutically Active Agent | APAP |
| Active Dose per tablet | 325 mg |
| % Moisture at End of Spraying (LOD at 105C) | 1.297 |
| % Moisture of Blend Before Sintering | 0.967 |
| Water Activity | 0.5546 |
| e' | 1.844 |
| e" | 0.0450 |
| Q value | 41 |
| Tablet Diameter (mm) | 12.5 |
| Tablet Weight (mg) | 596 |
| Power (W) | 2000 |
| Sintering Time (sec) | 1 |
| Oral Disintegration Time (sec) | <10 sec |
| Friability % (15 drops) | Not Tested |
| HEC: Hydroxyethyl cellulose<br>a: particles without HEC<br>b: could not be tested since these blends did not sinter to form a tablet<br>e' and e" tested on coated particles | |

## Example 4: Production of Coating Material for sintered Orally Disintegrating Tablets (sODT)

Coating Material Slurry:

1. The materials outlined in Table 2 were added to a suitable size vessel.

2. 500g of water was added and mixed until dissolved.

3. The slurry was then placed into an oven at 50°C and allowed to dry

4. The dried film was then milled into a powder. Material particle size was between No. 140 and 170 US Mesh Screens.

5. 0-3 percent of Silicon dioxide was blended to mixture from step #4 immediately prior to use.

Table 4

| Coating Materials for Dry Coating | | |
| --- | --- | --- |
| Material | % weight/weight | Batch Weight (g) |
| Soluplus® 1 | 50.0 | 50.0 |
| Starch | 43.0 | 43.0 |

(continued)

| Coating Materials for Dry Coating | | |
|---|---|---|
| Material | % weight/weight | Batch Weight (g) |
| Sucralose NF | 2.0 | 2.0 |
| Flavor | 2.0 | 2.0 |
| Titanium Dioxide | 3.0 | 3.0 |
| TOTAL | 100.0 | 100.0 |
| 1: Soluplus® Polyvinyl caprolactam polyvinyl acetate-polyethylene glycol graft co-polymer, commercially available from the BASF Corporation | | |

**Example 5: Production of Coating Material for sintered Orally Disintegrating Tablets (sODT)**

Coating Material Slurry:

1. The materials outlined in Table 5 were added to a suitable size vessel.
2. 500g of water was added and mixed until dissolved.
3. The slurry was then placed into an oven at 50°C and allowed to dry
4. The dried film was then milled into a powder. Material particle size was between No. 140 and 170 US Mesh Screens.
5. 0-3 percent of Silicon dioxide was blended with the mixture from step #4 immediately prior to use.

Table 5

| Coating Materials for Dry Coating | | |
|---|---|---|
| Material | % weight/weight | Batch Weight (g) |
| Precirol® ATO [1] | 50.0 | 50.0 |
| Starch | 48.5 | 48.5 |
| Titanium Dioxide | 1.5 | 1.5 |
| TOTAL | 100.0 | 100.0 |
| 1: Precirol® ATO - Commercially available from the Gattefosse Corporation | | |

Using the following powder coating composition of Example 5 and processing at 3000 Watts/1 second duration produced sintered tablet samples that showed material adherence to the electrodes surfaces, which is undesirable. Repeating the experiment at a lower power setting 2000 Watts/1 second duration, hence exposing the tablet coating layers to lower energy. The results at 2000 Watts/1 second produced samples which did not visibly adhere to the electrodes surfaces. Samples produced at the lower setting of 2000 Watts/1 second were later processed using radiant heat to coalesces the thin powder layer components stabilizing the tablet surface/tablet edge erosion while minimizing impact on the orally disintegration characteristic of the tablet.

**Example 6: Production of Coating Material for sintered Orally Disintegrating Tablets (sODT)**

Coating Material Slurry:

1. The materials outlined in Table 6 were added to a suitable size vessel.
2. 500g of water was added and mixed until dissolved.
3. The slurry was then placed into an oven at 50°C and allowed to dry
4. The dried film was then milled into a powder. Material particle size was between No. 140 and 170 US Mesh Screens.

5. 0-3 percent of Silicon dioxide (Syloid 63FP, commercially available from the Grace Corporation) was blended with the mixture from step #4 immediately prior to use to improve flow characteristics.

Table 6

| Coating Materials for Dry Coating | | |
|---|---|---|
| Material | % weight/weight | Batch Weight (g) |
| Kollidon® VA 64[1] | 71.0 | 71.0 |
| Lutrol F127[2] | 20.0 | 20.0 |
| Sucralose NF | 5.0 | 5.0 |
| Colorant | 1.0 | 1.0 |
| Titanium Dioxide | 3.0 | 3.0 |
| TOTAL | 100.0 | 100.0 |
| 1: Kollidon® VA64 vinylpyrrlidone-vinyl actetate copolymer, commercially available from the BASF Corporation 2: Lutrol: Commercially available from the BASF Corporation | | |

**Example 6A:** The process outlined in Example 3 was utilized to sinter orally disintegrating tablets with multiple powder layers.

**Example 6B:** The processed material from Table 6 was blended with an additional 30% by weight of Maltitol. This blend was the used to create an orally disintegrating tablet with multiple layers using the process outlined in Example 3.

**Example 7: Production of Coating Material for sintered Orally Disintegrating Tablets (sODT)**

Coating Material Blend:

1. The materials outlined in Table 7 were added to a suitable size heatable vessel.
2. 500g of water was added and mixed until dissolved.
3. The materials were heated to 80°C and melted and blended manually
4. The dried material was then milled into a powder. Material particle size was between No. 140 and 170 US Mesh Screens.
5. 0-3 percent of Silicon dioxide was blended to mixture from step #4 immediately prior to use.

Table 7

| Coating Materials for Dry Coating | | |
|---|---|---|
| Material | % weight/weight | Batch Weight (g) |
| Soluplus® [1] | 80.0 | 80.0 |
| Lutrol F127 | 20.0 | 20.0 |
| TOTAL | 100.0 | 100.0 |
| 1: Soluplus® Polyvinyl caprolactam polyvinyl acetate-polyethylene glycol graft co-polymer, commercially available from the BASF Corporation | | |

**Example 7A:** The process outlined in Example 3 was utilized to sinter orally disintegrating tablets with multiple powder layers.

### Example 8: Production of Coating for sintered Orally Disintegrating Tablets (sODT)

Blend: A dry base blend was prepared by manually blending the materials shown in Table 8. Additional materials were added to this base blend for the first powder layer as shown below.

Table 8

| Dry Blend Base using Pea Starch | | |
|---|---|---|
| Material | % weight/weight | Batch Weight (g) |
| Pea Starch | 96.0 | 96.0 |
| Colorant | 1.0 | 1.0 |
| Titanium Dioxide | 3.0 | 3.0 |
| TOTAL | 100.0 | 100.0 |

**Example 8A:** 100g of the Base Blend was subsequently manually blended with Lutrol® F127 at 10%, 20% and 30% of Lutrol® F127 by weight of the final blend

**Example 8B:** 100g of the Base Blend was subsequently manually blended with Precirol® ATO at 10%, 20% and 50% of Precirol® ATO by weight of the final blend

**Example 8C:** 100g of the Base Blend was subsequently manually blended with Polyethylene Glycol at 5%, 10%, and 20% of Polyethylene Glycol by weight of the final blend

**Example 8D:** 100g of the Base Blend was subsequently manually blended with Carnauba Wax at 10% of Carnauba Wax by weight of the final blend

Tablet process for Examples 8A, 8B, 8C, and 8D: The process outlined in Example 3 was utilized to sinter orally disintegrating tablets with multiple powder layers. Tablets were successfully sintered for each of these blends.

While the invention has been described above with reference to specific embodiments thereof, it is apparent that many changes, modifications, and variations can be made without departing from the inventive concept disclosed herein. Accordingly, it is intended to embrace all such changes, modifications, and variations that fall within the scope of the appended claims.

### Claims

1. A process for making a solid dosage form comprising at least one pharmaceutically active ingredient, the method comprising the steps of:

(i) providing a shape of said dosage form comprising a first powder layer comprising a surface stabilizing agent, a second powder layer comprising at least one activator and the pharmaceutically active ingredient, and a third layer comprising a second surface stabilizing agent, wherein the second layer is between the first layer and the third layer;

wherein, when measured according to the Slotted Line Method defined in the description, the activator has a Q value of less than 50 and the surface stabilizing agents each have a Q value of greater than 65; wherein the Q value is defined by the equation:

$$Q \text{ value} = e' / e''$$

where e" is the dielectric loss and e' is the dielectric constant;

(ii) applying radiofrequency energy for a sufficient period of time to the shape of step (i); and
(iii) applying heat for a sufficient period of time to the shape of step (i) to melt or soften the surface stabilizing agents.

2. The process of claim 1, wherein the second powder layer comprises particles having a substrate and the at least one activator; optionally wherein (i) the substrate has a Q value of greater than 100; or (ii) the particles have a lossy coating comprising the at least one activator.

3. The process of claim 1, wherein (i) the surface stabilizing agent has a melting point of from about 40 °C to about 200 °C;

   (ii) the surface stabilizing agent has a glass transition temperature of from about 20 °C to about 120 °C; or
   (iii) the surface stabilizing agent has a water solubility of from about 19 and about 48 MPa$^{1/2}$.

4. The process of claim 1, wherein the surface stabilizing agent is selected from the group consisting of polyethylene glycol, micronized crospovidone, vinylpyrrolidone-vinyl acetate copolymer, copovidone, xylitol, carbomer, PRECIROL ATO, polyethylene oxide, trehalose, and mixtures thereof.

5. The process of claim 1, wherein the surface stabilizing agent is vinylpyrrolidone-vinyl acetate copolymer.

6. The process of claim 1, wherein the surface stabilizing agent is from about 0.1 to about 10 percent, by weight of the dosage form.

7. The process of claim 1, further comprising the step of creating letters, numbers, indicia, and mixtures thereof on the dosage form by (i) applying, (ii) etching, or (iii) laser ablating, the letters, numbers, indicia, and mixtures thereof on or from the first layer.

8. The process of claim 1, wherein the second powder layer has an average particle size of less than 500 microns.

9. The process of claim 1, wherein said dosage form disintegrates in the mouth when placed on the tongue in less than about 30 seconds.

10. A process for making a dosage form comprising at least one pharmaceutically active ingredient, the method comprising the steps of:

    (i) introducing a first powder layer comprising a surface stabilizing agent, a second powder layer comprising at least one activator and the pharmaceutically active ingredient, and a third layer comprising a second surface stabilizing agent, wherein the second layer is between the first layer and the third layer, into a forming cavity within a die platen,

    wherein, when measured according to the Slotted Line Method defined in the description, the activator has a Q value of less than 50, and the surface stabilizing agents each have a Q value of greater than 65; wherein the Q value is defined by the equation:

$$Q \text{ value} = e' / e''$$

    where e" is the dielectric loss and e' is the dielectric constant;

    (ii) compacting the first powder layer, second powder layer and third powder layer by introducing at least one forming tool into the die platen with sufficient force such that the shape of the dosage form is formed;
    (iii) applying a radiofrequency energy for a sufficient period of time to the shape of step (ii);
    (iv) applying heat for a sufficient period of time to the shape of step (ii) to melt or soften the surface stabilizing agents; and
    (v) removing the dosage form from the forming cavity.

11. The process of claim 10, wherein step (v) is performed before step (iv).

12. The process of claim 10, wherein the process further comprises the step of cooling the dosage form in the die prior to removing the dosage form from the die.

13. A dosage form manufactured according to the process of claim 1.

**Patentansprüche**

1.  Verfahren zur Herstellung einer mindestens einen pharmazeutischen Wirkstoff umfassenden festen Verabreichungsform, wobei das Verfahren die folgenden Schritte umfasst:

    (i) die Bereitstellung einer Form der Verabreichungsform, die eine erste, ein Oberflächenstabilisierungsmittel umfassende Pulverschicht, eine zweite, mindestens einen Aktivator und den pharmazeutischen Wirkstoff umfassende Pulverschicht und eine dritte, ein zweites Oberflächenstabilisierungsmittel umfassende Schicht umfasst, wobei sich die zweite Schicht zwischen der ersten Schicht und der dritten Schicht befindet,

    wobei der Aktivator einen gemäß dem in der Beschreibung definierten Verfahren der geschlitzten Messleitung (Slotted Line) gemessenen Q-Wert von weniger als 50 aufweist und die Oberflächenstabilisierungsmittel jeweils einen Q-Wert von mehr als 65 aufweisen, wobei der Q-Wert durch die folgende Gleichung definiert ist:

    $$Q\text{-Wert} = e' \,/\, e''$$

    wobei e" der dielektrische Verlust und e' die Dielektrizitätskonstante ist;

    (ii) die Anwendung von Hochfrequenzenergie auf die Form von Schritt (i) über einen ausreichenden Zeitraum und (iii) die Anwendung von Wärme auf die Form von Schritt (i) über einen zum Schmelzen oder Erweichen der Oberflächenstabilisierungsmittel ausreichenden Zeitraum.

2.  Verfahren nach Anspruch 1, wobei die zweite Pulverschicht Partikel mit einem Substrat und dem mindestens einen Aktivator umfasst, wobei (i) das Substrat gegebenenfalls einen Q-Wert von größer als 100 aufweist oder (ii) die Partikel eine den mindestens einen Aktivator umfassende verlustbehaftete Beschichtung aufweisen.

3.  Verfahren nach Anspruch 1, wobei (i) das Oberflächenstabilisierungsmittel einen Schmelzpunkt von etwa 40 °C bis etwa 200 °C aufweist,

    (ii) das Oberflächenstabilisierungsmittel eine Glasübergangstemperatur von etwa 20 °C bis etwa 120 °C aufweist oder
    (iii) das Oberflächenstabilisierungsmittel eine Wasserlöslichkeit von etwa 19 bis etwa 48 MPa$^{1/2}$ aufweist.

4.  Verfahren nach Anspruch 1, wobei das Oberflächenstabilisierungsmittel aus der aus Polyethylenglykol, mikronisiertem Crospovidon, Vinylpyrrolidon-Vinylacetat-Copolymer, Copovidon, Xylitol, Carbomer, PRECIROL ATO, Polyethylenoxid, Trehalose und Mischungen davon bestehenden Gruppe ausgewählt ist.

5.  Verfahren nach Anspruch 1, wobei das Oberflächenstabilisierungsmittel Vinylpyrrolidon-Vinylacetat-Copolymer ist.

6.  Verfahren nach Anspruch 1, wobei das Oberflächenstabilisierungsmittel etwa 0,1 bis etwa 10 Gew.-% der Verabreichungsform ausmacht.

7.  Verfahren nach Anspruch 1, weiterhin umfassend den Schritt des Erzeugens von Buchstaben, Zahlen, Zeichen und Mischungen davon auf der Verabreichungsform durch (i) Aufbringen, (ii) Ätzen oder (iii) Laserabtragen der Buchstaben, Zahlen, Zeichen und Mischungen davon auf oder von der ersten Schicht.

8.  Verfahren nach Anspruch 1, wobei die zweite Pulverschicht eine durchschnittliche Teilchengröße von weniger als 500 Mikron aufweist.

9.  Verfahren nach Anspruch 1, wobei die Verabreichungsform in weniger als etwa 30 Sekunden im Mund zerfällt, wenn sie auf die Zunge gelegt wird.

10. Verfahren zur Herstellung einer mindestens einen pharmazeutischen Wirkstoff umfassenden Verabreichungsform, wobei das Verfahren die folgenden Schritte umfasst:

    (i) das Einbringen einer ersten, ein Oberflächenstabilisierungsmittel umfassenden Pulverschicht, einer zweiten, mindestens einen Aktivator und den pharmazeutischen Wirkstoff umfassenden Pulverschicht und einer dritten, ein zweites Oberflächenstabilisierungsmittel umfassenden Schicht, wobei sich die zweite Schicht zwischen der ersten Schicht und der dritten Schicht befindet, in einen Formhohlraum innerhalb einer Formplatte,

wobei der Aktivator einen gemäß dem in der Beschreibung definierten Verfahren der geschlitzten Messleitung (Slotted Line) gemessenen Q-Wert von weniger als 50 aufweist und die Oberflächenstabilisierungsmittel jeweils einen Q-Wert von mehr als 65 aufweisen, wobei der Q-Wert durch die folgende Gleichung definiert ist:

$$\text{Q-Wert} = e' / e''$$

wobei e" der dielektrische Verlust und e' die Dielektrizitätskonstante ist;

(ii) Verdichten der ersten Pulverschicht, der zweiten Pulverschicht und der dritten Pulverschicht durch Einführen mindestens eines Formwerkzeugs in die Formplatte mit einer zur Ausbildung der Form der Verabreichungsform ausreichenden Kraft,
(iii) die Anwendung einer Hochfrequenzenergie über einen ausreichenden Zeitraum auf die Form von Schritt (ii),
(iv) die Anwendung von Wärme auf die Form von Schritt (ii) über einen zum Schmelzen oder Erweichen der Oberflächenstabilisierungsmittel ausreichenden Zeitraum und
(v) das Entnehmen der Verabreichungsform aus dem Formhohlraum.

11. Verfahren nach Anspruch 10, wobei Schritt (v) vor Schritt (iv) durchgeführt wird.

12. Verfahren nach Anspruch 10, wobei das Verfahren weiterhin den Schritt des Kühlens der Verabreichungsform in der Form vor dem Entnehmen der Verabreichungsform aus der Form umfasst.

13. Verabreichungsform, hergestellt nach dem Verfahren nach Anspruch 1.


**Revendications**

1. Procédé pour préparer une forme posologique solide comprenant au moins un ingrédient pharmaceutiquement actif, le procédé comprenant les étapes de :

(i) fourniture d'une forme de ladite forme posologique comprenant une première couche de poudre comprenant un agent de stabilisation de surface, une deuxième couche de poudre comprenant au moins un activateur et l'ingrédient pharmaceutiquement actif, et une troisième couche comprenant un deuxième agent de stabilisation de surface, la deuxième couche se trouvant entre la première couche et la troisième couche ;

dans lequel, lorsqu'il est mesuré selon le procédé des lignes rainurées défini dans la description, l'activateur a une valeur Q inférieure à 50 et les agents de stabilisation de surface ont chacun une valeur Q supérieure à 65 ; la valeur Q étant définie par l'équation :

$$\text{valeur Q} = e' / e''$$

où e" est la perte diélectrique et e' est la constante diélectrique ;

(ii) application d'une énergie de radiofréquence pendant une durée suffisante à la forme de l'étape (i) ; et
(iii) application de chaleur pendant une durée suffisante à la forme de l'étape (i) pour faire fondre ou ramollir les agents de stabilisation de surface.

2. Procédé selon la revendication 1, dans lequel la deuxième couche de poudre comprend des particules ayant un substrat et l'au moins un activateur ; éventuellement, dans lequel (i) le substrat a une valeur Q supérieure à 100 ; ou (ii) les particules ont un revêtement avec perte comprenant l'au moins un activateur.

3. Procédé selon la revendication 1, dans lequel (i) l'agent de stabilisation de surface a un point de fusion d'environ 40 °C à environ 200 °C ;

(ii) l'agent de stabilisation de surface a une température de transition vitreuse d'environ 20 °C à environ 120 °C ; ou
(iii) l'agent de stabilisation de surface a une solubilité dans l'eau d'environ 19 à environ 48 MPa$^{1/2}$.

4. Procédé selon la revendication 1, dans lequel l'agent de stabilisation de surface est choisi dans le groupe constitué par le polyéthylène glycol, la crospovidone micronisée, un copolymère vinylpyrrolidone-acétate de vinyle, la copovidone, le xylitol, un carbomère, le PRECIROL ATO, un poly(oxyde d'éthylène), le tréhalose et leurs mélanges.

5. Procédé selon la revendication 1, dans lequel l'agent de stabilisation de surface est un copolymère vinylpyrrolidone-acétate de vinyle.

6. Procédé selon la revendication 1, dans lequel l'agent de stabilisation de surface représente environ 0,1 à environ 10 % en poids de la forme posologique.

7. Procédé selon la revendication 1, comprenant en outre l'étape de création de lettres, de nombres, de signes distinctifs et de mélanges correspondants sur la forme posologique en (i) appliquant, (ii) gravant, ou (iii) ablatant au laser, les lettres, les nombre, les signes distinctifs et leurs mélanges sur ou à partir de la première couche.

8. Procédé selon la revendication 1, dans lequel la deuxième couche de poudre a une taille moyenne de particule inférieure à 500 microns.

9. Procédé selon la revendication 1, dans lequel ladite forme posologique se désintègre dans la bouche lorsqu'elle est placée sur la langue en moins d'environ 30 secondes.

10. Procédé pour préparer une forme posologique comprenant au moins un ingrédient pharmaceutiquement actif, le procédé comprenant les étapes de :

(i) introduction d'une première couche de poudre comprenant un agent de stabilisation de surface, d'une deuxième couche de poudre comprenant au moins un activateur et l'ingrédient pharmaceutiquement actif, et d'une troisième couche comprenant un deuxième agent de stabilisation de surface, la deuxième couche étant entre la première couche et la troisième couche, dans une cavité de mise en forme à l'intérieur d'un plateau de matrice,

dans lequel, lorsqu'il est mesuré selon le procédé des lignes rainurées défini dans la description, l'activateur a une valeur Q inférieure à 50, et les agents de stabilisation de surface ont chacun une valeur Q supérieure à 65 ; la valeur Q étant définie par l'équation :

$$\text{valeur Q} = e' / e''$$

où e'' est la perte diélectrique et e' est la constante diélectrique ;

(ii) compactage de la première couche de poudre, de la deuxième couche de poudre et de la troisième couche de poudre par introduction d'au moins un outil de mise en forme dans le plateau de matrice avec une force suffisante pour que la forme de la forme posologique soit mise en forme ;
(iii) application d'une énergie de radiofréquence pendant une durée suffisante à la forme de l'étape (ii) ;
(iv) application de chaleur pendant une durée suffisante à la forme de l'étape (ii) pour faire fondre ou ramollir les agents de stabilisation de surface ; et
(v) élimination de la forme posologique de la cavité de mise en forme.

11. Procédé selon la revendication 10, dans lequel l'étape (v) est effectuée avant l'étape (iv).

12. Procédé selon la revendication 10, dans lequel le procédé comprend en outre l'étape de refroidissement de la forme posologique dans la matrice avant d'éliminer la forme posologique de la matrice.

13. Forme posologique fabriquée selon le procédé de la revendication 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5464632 A
- US 5223264 A
- US 5178878 A
- US 6589554 B
- US 6224905 B
- US 2013295174 A1
- US 2011070286 A1
- US 2013295211 A1
- WO 2015105992 A1
- US 8871263 B
- US 9610224 B
- US 8807979 B
- US 8343533 B
- US 8784781 B
- US 8858210 B
- US 9233491 B
- US 9445971 B
- US 9511028 B
- US 9789066 B
- US 20130295174 A
- US 20110070286 A
- US 20110318411 A
- US 20110319492 A
- US 4851226 A
- US 5075114 A
- US 5489436 A
- US 20040156902
- US 6767200 B
- US 20110068511 A
- US 20130295211 A

**Non-patent literature cited in the description**

- Design of Prodrugs. Elsevier, 1985